# EUROPÄISCHE PATENTANMELDUNG

(11) **EP 1 371 729 A2**
(43) Veröffentlichungstag der Anmeldung: **17.12.2003**
(21) Anmeldenummer: 03090080.7
(22) Anmeldetag: 25.03.2003
(51) Int. Cl.: C12N 15/16, C07K 14/575, G01N 33/574, A61K 38/22, A61P 15/00, A61P 35/00

(54) **Verwendungen von TFF3 bindenden Substanzen zur Diagnose und Behandlung von Krebserkrankungen**

(30) Priorität: 02.04.2002 DE 10215320
(71) Anmelder: metaGen Pharmaceuticals GmbH, 13347 Berlin (DE)
(72) Erfinder: Lehmann, Kerstin, 13347 Berlin (DE)
(74) Vertreter: Jungblut, Bernhard Jakob, Dr.

(57) **Zusammenfassung**

Die Erfindung betrifft Verwendungen von TFF3 zur Diagnose und Behandlung von Uterus- und/oder Ovartumoren sowie zum Screenen nach Substanzen für solche Zwecke.

## Beschreibung

### Gebiet der Erfindung

Die Erfindung betrifft neue Verwendungen von TFF3 oder daraus abgeleiteten Sequenzen zum Screenen nach daran bindenden Substanzen, sowie die Verwendung von an TFF3 bindenden Substanzen zur Diagnose und/oder Behandlung von Tumor-Erkrankungen.

### Hintergrund der Erfindung und Stand der Technik

Die Sequenz von TFF3 ist bekannt unter der AcNo XM_032969.

TFF3 gehört zu der Familie der sogenannten trefoil factor peptides, welche die 3 Mitglieder TFF1, TFF2 und TFF3 umfaßt. TFF3 weist ein Motiv mit drei intraketten-Loops auf, welche durch Disulfidbrücken gebildet werden, wodurch TFF3 eine Resistenz gegen Proteaseverdau verliehen wird. TFF3 wird von Epithelzellen im menschlichen Dickdarm sowie Dünndarm gebildet und apical sekretiert. Sekretiertes TFF3, welches motogene Eigenschaften besitzt, spielt in diesen Darmzellen eine wichtige Rolle, z.B. in der Homeostase und in der Reepithelialisierung bei Verletzungen des Darmoberflächengewebes. Für eine Übersicht wird auf die Literaturstelle W.M. Wong et al., Gut. 44:890-895 (1999) verwiesen.

Gemäß der Literaturstelle R.J. Longman et al., Br J Surg 86:740-748 (1999) ist TFF3 in Dickdarm- und Brusttumoren hochreguliert und trägt zum Krankheitsfortschritt bei. Gemäß F.E.B. May et al., J of Pathology 182:404-413 (1997) ist TFF3 Expression auch in Brusttumoren in einer hormonabhängigen Weise induziert. Verschiedene Publikationen beschäftigen sich mit der motogenen, pro-invasiven sowie anti-apoptotischen Wirkung von TFF3. Der Wirkmechanismus von TFF3 ist noch nicht vollständig geklärt. Gemäß verschiedener Literaturstellen kann TFF3 in autocriner Weise wirken, allerdings ist ein entsprechender Oberflächen-Rezeptor für TFF3 noch nicht identifiziert worden (Emami et al., 2001, Podolsky, 2000).

Verschiedene Patentpublikationen betreffen TFF3. Dies sind beispielsweise US-5,733,748, US-6,063,755, US-6,221,840, WO-01/00828, WO-01/25272, WO-01/34802, WO-01/46475, WO-99/10377, WO-99/47669 und WO-99/47374. In diesen Literaturstellen wird TFF3 in Verbindung gebracht mit inflammatorischen Magenerkrankungen, Erkrankungen des Gastrointestinaltraktes, Dickdarmkrebs, Lungenkrebs und Prostatkrebs. Teilweise wird TFF3 als Target für Antikörper beschrieben.

Uterus- und Ovarkrebs sind eine mit zunehmendem Alter mit beachtlicher Inzidenz auftretende Erkrankung. Bislang werden diese Tumorerkrankungen im wesentlichen pathologisch diagnostiziert und meist durch Entfernung behandelt. Die Entfernung von Organen hat verschiedene nachteilige Effekte auf eine Patientin, insbesondere den Verlust der Gebärfähigkeit. Eine verbesserte Diagnose und Behandlung dieser Krebsarten, insbesondere ohne das Erfordernis einer Entfernung der Organe, ist daher in hohem Maße wünschenswert.

Darüber hinaus findet im fortgeschrittenen Tumorstadium häufig eine Verbreitung des Ovarialcarcinoms in den Peritonealraum statt. Nach der Entfernung der Organe (Ovarien) verbleiben diese gestreuten Tumore im Peritonealgewebe. Die Diagnose und Behandlung dieser post-operativen rezidiven Ovarialtumore ist daher von größtem Interesse.

### Technisches Problem der Erfindung

Der Erfindung liegt das technische Problem zugrunde, pharmazeutische Zusammensetzungen zur Diagnose und/oder zur Behandlung von Uterus- und/oder Ovarkrebserkrankungen anzugeben sowie Mittel zu deren Identifizierung.

### Grundzüge der Erfindung sowie bevorzugte Ausführungsbeispiele.

Die Erfindung lehrt die Verwendung einer für TFF3 codierenden Nukleinsäure und/oder eines TFF3 Peptids oder Proteins zur Detektion von Uterus- und/oder Ovartumoren oder zur Detektion eines Risikos der Erkrankung an Uterusund/oder Ovartumoren, wobei eine Uterus- oder Ovar-Gewebeprobe auf Überexpression von TFF3 RNA oder auf Überexpression eines TFF3 Proteins untersucht wird. Eine an für TFF3 codierende Nukleinsäure oder eine an TFF3 Protein oder Peptid bindende Detektorsubstanz, vorzugsweise enthaltend eine Reportergruppe, kann verwendet werden, wobei Bindung besagter Nukleinsäure und/oder besagten Proteins oder Peptids an die Detektorsubstanz halbquantitativ oder quantitativ detektiert wird.

Die Erfindung lehrt weiterhin die Verwendung einer TFF3 RNA oder eines TFF3 Proteins oder Peptids zum Screenen nach daran bindenden Substanzen, insbesondere prospektiven Wirkstoffen zur Inhibierung von besagter RNA oder besagtem Protein oder Peptid oder prospektiven Detektorsubstanzen, wobei eine prospektive Substanz oder eine Mischung solcher prospektiver Substanzen mit besagter RNA oder besagtem Protein oder Peptid kontaktiert wird, wobei mit einem Bindungsassay Bindungsereignisse festgestellt werden, und wobei eine bindende prospektive Substanz, ggf. nach Dekonvolutierung, selektiert wird.

Die Erfindung lehrt schließlich die Verwendung einer TFF3 inhibierenden oder daran bindenden Substanz zur Herstellung einer pharmazeutischen Zusammensetzung zur Behandlung von Uterus- und/oder Ovartumoren. Die Substanz kann ein Antikörper sein, welcher durch Immunisierung eines nicht-menschlichen Säugetiers mit einem TFF3 Peptid oder Protein erhältlich ist. Als Immunogene können auch mit der cDNA von TFF3 transient oder stabil transfizierte Tumorzelllinien, NIH3T3 Zellen, endogen TFF3 exprimierende Tumorzellen (z.B. BT474 oder OV90), rekombinant in Insektenzellen hergestelltes TFF3 oder die TFF3 cDNA (in nicht-menschlichen Säugetieren) verwendet werden. Die Substanz kann aber auch eine Mimikryverbindung eines Antikörpers gegen ein TFF3 Peptid oder Protein sein. Die Substanz kann schließlich ein Aptamer, eine antisense RNA, eine RNAi, oder ein Ribozym sein. Die Substanz kann zusätzlich eine zytotoxische und/oder immunstimulierende Komponente tragen. Die pharmazeutische Zusammensetzung kann zur lokalen Applikation in Tumorzellen enthaltendem Gewebe hergerichtet sein.

Die Erfindung läßt sich im Rahmen eines Verfahrens zur Diagnose einer Uterus-, und/oder Ovartumorerkrankung verwenden, wobei eine Detektorsubstanz in einer Ausführungsform mit einer Reportergruppe in zu untersuchendes Gewebe appliziert wird, wobei das zu untersuchende Gewebe dann einer Detektionsverfahrensstufe unterworfen wird, welche sensitiv für die Reportergruppe ist, und wobei im Fall der Detektion eines definierten Mindestwertes der Reportergruppe im Gewebe das Gewebe als Tumorzellen enthaltend qualifiziert wird, sowie eines Verfahrens zur Behandlung einer Uterus- und/oder Ovartumorerkrankung, wobei eine erfindungsgemäße pharmazeutische Zusammensetzung in einer physiologisch wirksamen Dosis einem Patienten dargereicht wird.

Die Erfindung beruht auf der Erkenntnis, daß TFF3 überexprimiert ist bzw. differenziell exprimiert wird in Uterus- und Ovartumoren, i.e. in besagten Tumorgeweben ist die Expression höher bzw. überhaupt zu beobachten, verglichen mit normalen Zellen gleichen Gewebes, und der daraus herleitbaren technische Lehre, daß TFF3 als Zielmolekül bei der Diagnostik und Therapie dieser Erkrankungen eingesetzt werden kann. TFF3 kann also als Marker zur Identifizierung von Tumorzellen in den besagten Tumorgeweben dienen. Auf der anderen Seite bietet die Inhibierung von TFF3, insbesondere auch bei lokaler Applikation, die Möglichkeit, in die Tumor-spezifischen TFF3 Assoziationen mit anderen Prozessen in den Tumorzellen einzugreifen und somit letztendlich den tumorzellenspezifisch veränderten Stoffwechsel zu stören und zu einem Absterben oder zumindest einer Wachstumshemmung der Tumorzellen beizutragen. Die Inhibierung von TFF3 kann auch in Kombination mit einer Chemo- oder Radiotherapie eingesetzt werden.

In diesen Zusammenhängen ist auch wichtig zu verstehen, dass die Erkenntis der Überexpression allein nicht Basis der Erfindung ist. Vielmehr ist TFF3 aus einem Pool hochregulierter Gene identifiziert worden, welches unmittelbar tumorrelevant ist. Denn grundsätzlich akkumulieren Krebszellen diverseste Änderungen in Expressionmustern, wie beispielsweise Erhöhung der Genkopiezahl, Erhöhung der Proteinmenge, Anschalten von stromabwärts in der Signaltransduktionskaskade wirkenden Genen durch zu hohe Proteinmengen (Triggereffekt), usw. Viele dieser Änderungen sind jedoch oft eher Folge der Tumorgenese und nicht notwendigerweise auch ursächlich daran beteiligt. TFF3 wurde jedoch als ein Gen identifiziert, welches die Tumorgenese essentiell beeinflußt und folglich kausal tumorrelevant ist. Denn TFF3 besitzt antiapoptotische Wirkung, i.e. Überproduktion von TFF3 Tumorzellen schützt diese vor (ansonsten natürlicherweise stattfindende) Apoptose und führt letztendlich zur Progression des Tumors. Hierzu wird ergänzend auf die Beispiele verwiesen.

Im Rahmen der Efindung kann es sich empfehlen, im Vorfeld einer Behandlung mit einer erfindungsgemäßen pharmazeutischen Zusammensetzung eine Probe aus einem Gewebe, welches als Tumorgewebe mit anderen Methoden identifiziert ist, zu entnehmen und die Gewebeprobe auf Expression bzw. Überexpression von TFF3 zu untersuchen. Alternativ kann mit einer erfindungsgemäßen Detektorsubstanz zur Diagnose in vivo auf TFF3 Abhängigkeit getestet werden. Wird eine Expression bzw. Überexpression von TFF3 gegenüber Normalgewebe gleichen Typs bzw. überhaupt eine Expression festgestellt, so ist die Anwendung der erfindungsgemäßen pharmazeutischen Zusammensetzung indiziert.

Handelt es sich bei dem Tumor um einen Typus, bei welchem Tumorzellen TFF3 exprimieren, Normalzellen gleichen Gewebetyps jedoch nicht, so ist es besonders bevorzugt, wenn die an TFF3 bindende Substanz zusätzlich eine zytotoxische, radioaktive und/oder immunstimulierende Komponente trägt. Dies führt dann letztendlich dazu, dass praktisch ausschließlich Tumorzellen getötet werden, sei es durch die Zytotoxizität, sei es durch Angriff durch das stimulierte Immunsystem, während Normalzellen in dem Gewebe praktisch vollständig erhalten bleiben. In dieser Ausführungsform braucht die bindende Substanz selbst nicht inhibierend auf TFF3 zu wirken, da die bindende Substanz dann lediglich als Marker funktionieren muß, welcher die Komponenten zu Ziel-Tumorzellen trägt. Im Falle des Einsatzes einer zytotoxischen, radioaktiven und/oder immunstimulierenden Komponente wird es sich besonders empfehlen, wenn die pharmazeutische Zusammensetzung zur lokalen Applikation in Tumorzellen enthaltendem Gewebe hergerichtet ist, beispielsweise zur Injektion.

### Definitionen.

Im Rahmen dieser Beschreibung wird die Bezeichnung TFF3 als Oberbegriff für alle humanen Isoformen, Splice Varianten und regulatorische RNA, bekannt oder neu, auf Nukleinsäuren- oder Aminosäurenbasis, verwendet. Mit diesen Begriffen mit umfaßt sind auch die ggf. im Rahmen dieser Beschreibung offenbarten kurzen Sequenzen, welche aus den verschiedenen Varianten stammen, beispielsweise Immunisierungssequenzen. Weiterhin mit umfaßt sind auch Homologe, wobei die Homologie zumindest 80%, vorzugsweise mehr als 90%, höchstvorzugsweise mehr als 95%, beträgt (berechnet mit dem Programm MEGALIGN, DNASTAR LASERGENE, in der zum nmeldezeitpunkt gültigen Fassung). Im Falle der Nukleinsäuresequenzen sind auch komplementäre oder allelische Varianten mit umfaßt. Weiterhin sind Sequenzen umfaßt, welche lediglich Teilsequenzen, beispielsweise ein Exon oder mehrere Exons, der explizit offenbarten Sequenzen oder komplementärer Sequenzen hierzu darstellen, mit der Maßgabe, daß diese Teilsequenzen im Falle der Nukleinsäuren eine für eine Hybridisierung mit einer erfindungs-gemäßen Nukleinsäure hinreichende Länge, zumindest 50 oder 150 Basen, aufweisen und im Falle der Proteine bzw. Peptide, ggf. codiert durch eine Nukleinsäure, mit zumindest gleicher Affinität an ein protein- oder peptidspezifisches Zielmoleküle binden. Weiterhin sind alle mit erfindungs-gemäß eingesetzten Nukleinsäuren hybridisierende Nukleinsäuren umfasst, nämlich solche, die unter stringenten Bedingungen (5°C bis 25°C unterhalb der Aufschmelztemperatur; siehe ergänzend J.M. Sambrook et al., A laboratory manual, Cold Spring Harbor Laboratory Press (1989) und E.M. Southern, J Mol Biol, 98:503ff (1975)) hybridisieren. Es versteht sich, daß die Erfindung auch Expressionskassetten umfaßt, i.e. eine oder mehrere der erfindungsgemäßen Nukleinsäuresequenzen mit mindestens einer Kontroll- oder regulatorischen Sequenz. Eine solche Ex-pressionskassette kann auch eine Sequenz für ein bekanntes Protein umfassen, wobei im Zuge der Translation ein Fusionsprotein aus einem bekannten Protein und einem erfindungsgemäßen Protein oder Peptid entsteht. Ebenso sind auch antisense Sequenzen zu den vorstehenden Nukleinsäuresequenzen umfaßt. Mit umfaßt sind auch Expressionsvektoren enthaltend erfindungsgemäße Nukleinsäuren sowie damit transformierte Wirtszellen. Schließlich sind RNA sowie damit korrelierende DNA und umgekehrt umfaßt, ebenso wie genomische DNA als auch korrelierte cDNA und umgekehrt.

Im Zusammenhang mit erfindungsgemäßen Verwendungen umfassen die Begriffe der Nukleinsäuren oder Proteine bzw. Peptide neben den Volllängen der offenbarten Sequenzen (siehe auch vorstehender Absatz) auch Teilsequenzen hieraus, und zwar mit einer Mindestlänge von 12 Nukleotiden, vorzugsweise einer Mindestlänge von 30 bis 90 Nukleotiden, im Falle der Nukleisäuren und einer Mindestlänge von 4 Aminosäuren, vorzugsweise einer Mindestlänge von 10 bis 30 Aminosäuren, im Falle der Proteine oder Peptide.

Die Begriffe der Detektion und/oder der Behandlung umfassen optional auch die Detektion und/oder Behandlung von Metastasen aus Primärtumoren in sonstigen Geweben. Der Begriff der Behandlung umfasst auch die Prophylaxe sowie die Nachbehandlung (bei nicht mehr detektierbarem Tumor oder bei stabilem Tumor). Der Begriff der Prophylaxe umfasst im Zusammenhang mit der Detektion auch die Vorsorgeuntersuchung.

Als Inhibitor ist eine Verbindung oder Substanz bezeichnet, welche entweder die Bildung von TFF3 inhibiert oder gebildetes TFF3 in der Aktivität reduziert, bezogen auf die TFF3 in vitro oder in vivo Aktivität in Abwesenheit des Inhibitors. Insofern kann ein Inhibitor einerseits eine Substanz sein, welche in der Entstehungskaskade von TFF3 inhibierend eingreift. Auf der anderen Seite kann ein Inhibitor eine Substanz sein, welche mit gebildetem TFF3 eine Bindung eingeht, und zwar dergestalt, dass weitere physiologische Wechselwirkungen mit endogenen Substanzen zumindest reduziert sind. Als Inhibitoren können Moleküle dienen, die die Transkription des Zielgens beeinflussen und inhibieren. Solche Moleküle können Polyamide oder Zinkfingerproteine sein, die durch Bindung an DNA-Regionen der basalen Transkriptionsmaschinerie die Transkription verhindern. Die Transkription kann indirekt auch über die Inhibierung von Transkriptionsfaktoren geschehen, die für die Transkription des Zielgens essentiell sind. Die Inhibierung solcher Transkriptionsfaktoren kann über die Bindung an sogenannte Decoy-Aptamere gewährleistet werden.

Mimikry-Moleküle sind Verbindungen, die den variablen Bereich, insbesondere den Bindungsbereich eines Antikörpers nachbilden und an gleicher Stelle eines Zielmoleküls binden wie der zu Grunde liegende Antikörper. Desweiteren fallen Anticaline und Affibodies unter den Begriff der Mimikrimoleküle.

Der Begriff der Antikörper umfaßt humane, humanisierte und nicht-humanisierte polyklonale oder monoklonale Antikörper (typischerweise in vivo hergestellt). Der Begriff umfasst desweiteren Phage-Display-Antikörper Ribozym-Display Antikörper (kovalente Fusion zwischen RNA und Protein) und RNA-Display Antikörper (in vitro hergestellt). Der Begriff umfasst auch Antikörper, welche durch Chimerisierung, Hu-manisierung oder De-Imunisierung (Ausschneiden von T-Zellepitopen aus dem humanen Antikörper, die unerwünschte Immunreaktionen auslösen) modifiziert sind, sowie spezifische Fragmente der leichten und/oder der schweren Kette des variablen Bereiches zu Grunde liegender Antikörper vorstehender Art. Die Herstellung bzw. Gewinnung solcher Antikörper mit vorgegebenen Immunogenen ist dem Durchschnittsfachmann wohl vertraut und braucht nicht näher erläutert zu werden. Weiterhin umfaßt sind bispezifische Antikörper, welche einerseits an ein Auslösemolekül einer Immun-Effektorzelle (z.B. CD3, CD16, CD64) und andererseits an ein Antigen der Tumorzielzelle binden. Dies bewirkt letzendlich im Bindungsfall, daß die Tumorzelle getötet wird.

Die galenische Herrichtung einer erfindungsgemäßen pharmazeutischen Zusammensetzung kann in fachüblicher Weise erfolgen. Als Gegenionen für ionische Verbindungen kommen beispielsweise Na⁺, K⁺, Li⁺ oder Cyclohexylammonium infrage. Geeigente feste oder flüssige galenische Zubereitungsformen sind beispielsweise Granulate, Pulver, Dragees, Tabletten, (Mikro-) Kapseln, Suppositorien, Sirupe, Säfte, Suspensionen, Emulsionen, Tropfen oder Lösungen zur Injek-tion (i.v., i.p., i.m., s.c.) oder Vernebelung (Aerosole), transdermale Systeme, sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als Hilfsstoffe sei Magnesiumcarbonat, Titandioxid, Lactose, Mannit und andere Zucker, Talcum, Milcheiweiß, Gelatine, Stärke, Zellulose und ihre Derivate, tierische und pflanzliche Öle wie Lebertran, Sonnenblumen-, Erdnussoder Sesamöl, Polyethylenglycole und Lösungsmittel, wie etwa steriles Wasser und ein- oder mehrwertige Alkohole, beispielsweise Glycerin, genannt. Eine erfindungsgemäße pharmazeutische Zusammensetzung ist dadurch herstellbar, dass mindestens ein erfindungsgemäß verwendeter TFF3-Inhibitor in definierter Dosis mit einem pharmazeutisch geeigneten und physiologisch verträglichen Träger und ggf. weiteren geeigneten Wirk-, Zusatz- oder Hilfsstoffen mit definierter Inhibitordosis gemischt und zu der gewünschten Darreichungsform hergerichtet ist.

Tumorzellen exprimieren TFF3 differenziell, wenn Normal-5 zellen des gleichen Gewebetyps dieses nicht exprimieren. Tumorzellen überexprimieren TFF3 spezifisch bzw. differenziell, wenn TFF3 im Vergleich zu Normalzellen des gleichen Gewebes zumindest in doppelter Menge exprimiert wird.

Zytotoxische Komponenten bzw. Gruppen sind Verbindungen, welche direkt oder indirekt Apoptose einleiten oder zumindest wachstumshemmend wirken. Solche Gruppen bzw. Verbindungen können neben Radioisotopen (z.B. 188Re, 213Bi, 99mTc, 90Y, 131J, 177Lu) insbesondere Toxine (z.B. Diphterie-Pseudomonas-Toxin) oder Zytostatika einschließlich sogenannter Prodrugs sein, welche in der Tumortherapie eingesetzt werden. Beispiele hierfür sind: Alkylantien (z.B. Mechlorethamin, Ifosfamid, Chlorambucil, Cyclophosphamid, Melphalan, Alkylsulfonate, Busulphan, Nitrosoharnstoffe, Carmustin, Lomustin, Semustin, Triazene, Dacarbazin), Antimetaboliten (z.B. Folsäure-Antagonisten, Methotrexat, Pyrimidin-Analoga, Fluoruracil, Fluordesoxyuridin, Cytarabin, Gemcitabin, Purin-Analoga, Mercaptopurin), Mitosehemmer (z.B. Vincaalkaloide, Vin-cristin, Vinblastin, Paclitaxal, Docetaxel, Protaxel), Epipodophyllotoxine (z.B. Etoposid, Teniposid), Antibiotika (z.B. Dactinomycin, Daunorubicin, Idarubicin, Anthracycline, Bleomycin, L-Asparaginase), Platinkomplexverbindungen (z.B. Cisplatin), Hormone und verwandte Verbindungen (z.B. Nebennierensindensteroide, Aminogluthetimid, Gestagene, Östrogene, Androgene, Antiöstrogene, Tamoxifen, Steriodanaloga, Flutamid). Bei Bindung einer solchen Verbindung mit einer an TFF3 bindenden Substanz erfolgt die Kopplung dergestalt, daß die Affinität zu TFF3 um nicht mehr als 90%, vorzugsweise 50%, bezogen auf die Substanz ohne zytostatische Gruppe, reduziert ist und die zytostatische Wirkung der Gruppe um nicht mehr als 90%, vorzugsweise 50%, bezogen auf die Verbindung ohne Substanz, reduziert ist.

Eine immunstimulierende Komponente ist meist ein Protein oder ein wirksamer Bestandteil hiervon, welches Zellen des Immunsystems stimuliert. Beispiele hierfür sind: Zytokine, wie M-CSF, GM-CSF, G-CSF, Interferone, wie IFN-alpha, beta, gamma, Interleukine wie IL-1 bis -16 (außer -8), human LIF, Chemokine wie Rantes, MCAF, MIP-1-alpha, -beta, NAP-1 und IL-8.

Eine Reportergruppe ist ein Atom, Molekül oder eine Verbindung, welche in Verbindung mit einem hierauf abgestellten Assay den Nachweis der Reportergruppe und der somit mit der Reportergruppe verbundenen Verbindung oder Substanz ermöglicht. Beispiele für Reportergruppen und hiermit assoziierte Detektionsmethoden sind: 32P-Labeling und Intensitätsmessung mittels Phosphoimager. Viele weitere Beispiele sind dem Durchschnittsfachmann bekannt und bedürfen nicht der detaillierten Aufzählung.

Eine an TFF3 bindende Substanz kann eine Substanz sein, welche ein TFF3-Protein oder TFF3-RNA bindet.

Im Rahmen der vorstehenden Definition gegenüber dem engen Wortsinn erweiterte Begriffsbestimmungen umfassen auch die bestimmten Begriffe im engen Wortsinn.

Vorbekannte Sequenzen und/oder deren Verwendung, welche unter die im Rahmen dieser Beschreibung verwendete Definitionen fallen, können durch einen Disclaimer in Patentansprüchen ausgeschlossen werden.

### Beispiele.

Im Folgenden wird die Erfindung anhand von lediglich bevorzugte Ausführungsformen darstellenden Beispielen und Figuren näher erläutert. Es zeigen:
- Fig. 1:: Cancer Profiling Array für TFF3,
- Fig. 2:: Quantifizierung des Befundes für Uterusproben,
- Fig. 3:: Quantifizierung des Befundes für Ovarproben,
- Fig. 4:: ein Hammerhead Ribozyme, welches TFF3 mRNA schneidet,
- Fig. 5:: RNA in situ Hybridisierung,
- Fig. 6:: TFF3 cDNA
- Fig. 7:: Immunhistochemischer TFF3 Nachweis,
- Fig. 8:: proteinchemischer Nachweis von TFF3 in Tumorzellen,
- Fig. 9:: Zusammenfassung der proteinchemischen Untersuchungen aus 16 Ovartumorgeweben,
- Fig. 10:: TFF3 Nachweis durch in situ Hybridisierung,
- Fig. 11 - 15:: Isolierung und Aufreinigung von TFF3.
- Fig. 16:: TFF3 Aminosäurensequenz sowie Teilsequenzen aus TFF3 (Aminosäuren sowie Nukleinsäuren), welche funktional wesentlich für TFF3 sind.
- Fig. 17:: antiapoptotische Wirkung von TFF3, und
- Fig. 18:: Reduktion von DNA-Fragmentierung durch TFF3 Überexpression.

### Beispiel 1: Überexpression in Uterus- und Ovartumor

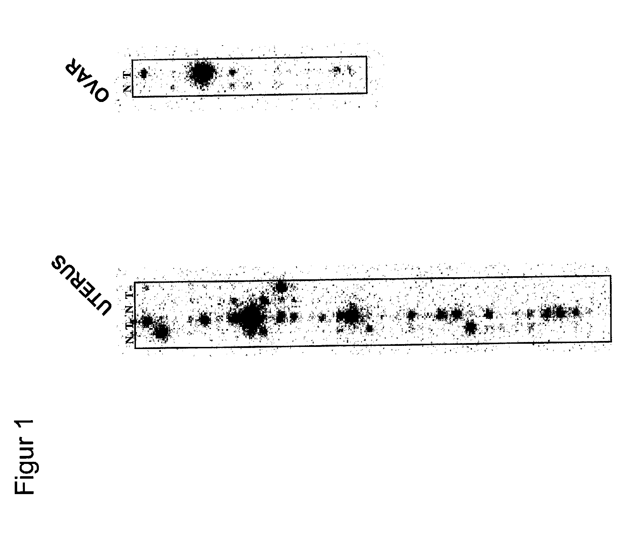

Ein Cancer Profiling Array (CPA, von Clontech erhalten) mit 241 aufgetragenen humanen cDNAs von Tumor- und korrespondierenden Normalgeweben wurde unter Verwendung einer 32P markierten TFF3-spezifischen Sonde analysiert. Die Figur 1 zeigt eine vergleichsweise starke Überexpression insbesondere in Uterus- und Ovartumoren.

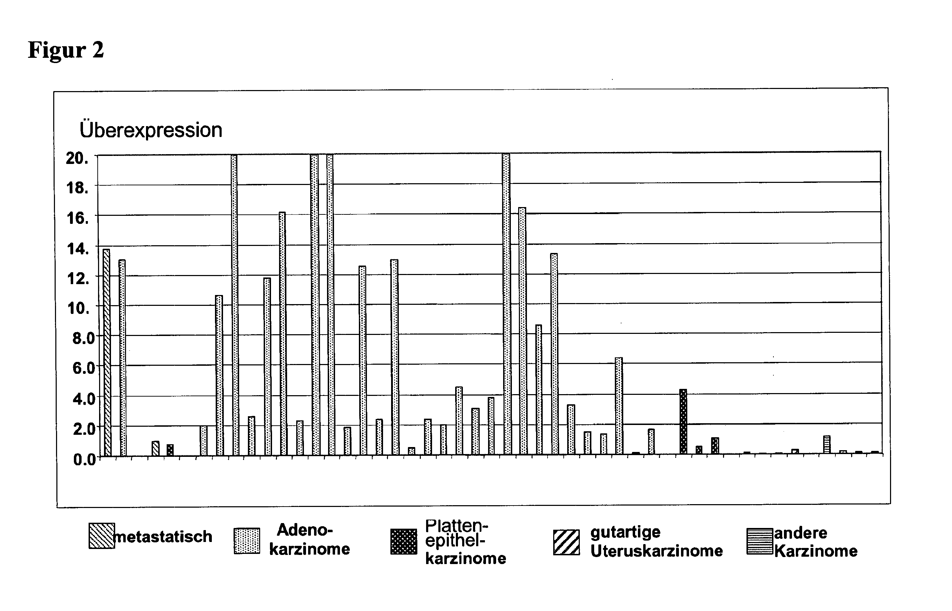

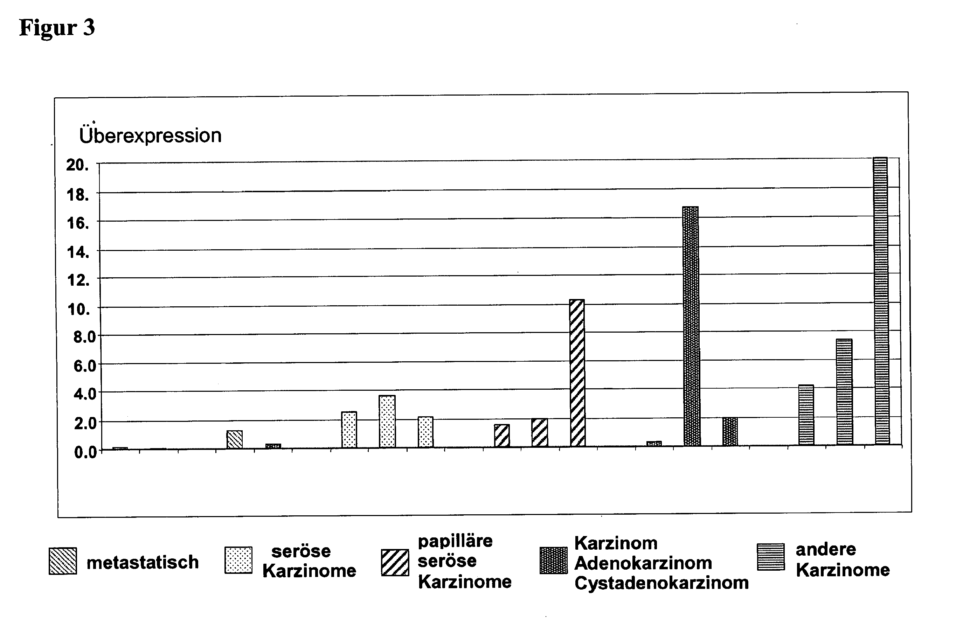

Die Ergebnisse der quantitativen Analyse des CPAs sind in den Figuren 2 und 3 dargestellt. Man erkennt, daß in 59% (26 aus 44 Patienten) der Uterustumoren die Expression von TFF3 um zumindest den Faktor 2 erhöht ist (Fig. 2). In der Figur 3 erkennt man, daß TFF3 in 62% (10 aus 16 Patienten) der Ovartumore um zumindest den Faktor 2 erhöht exprimiert wird.

### Beispiel 2: in-vivo Diagnostik von TFF3 mittels Antikörpern

In diesem Beispiel wird die Markierung eines Tumors bzw. seiner Metastasen durch einen anti-TFF3-Antikörper in vivo (Mausmodell) beschrieben. In NMRI-Nacktmäuse werden 2.000.000 TFF3-transfizierte humane Zellen oder endogen exprimierende Zellen transplantiert. 30 Tage nach der Transplantation wird den Mäusen markierter Antikörper injiziert. Die Kontrolltiere werden mit einem nicht relevanten Antikörper behandelt. Wenige Stunden nach der Antikörperapplikation werden die Tiere getötet und alle Organe entnommen. Es erfolgt die Messung der relativen Anreicherung in Metastasen bzw. anderen Organen. Die Anreicherung sollte überwiegend im Tumor erfolgen.

Bei den anti-TFF3 Antikörpern handelt es sich um monoklonale Antikörper gegen humanes TFF3 Protein, konjugiert mit einem Trägerprotein, in einem nicht-humanen Säugetier. Die Herstellung monoklonaler Antikörper ist dem Durchschnittsfachmann bekannt. Zur Immunisierung können TFF3 Peptide, rekombinantes TFF3 Protein sowie mit der cDNA von TFF3 transient oder stabil transfizierte NIH3T3 Zellen oder die TFF3 cDNA verwendet werden.

Beispiele für Immunisierungspeptide bzw. -sequenzen sind CAVPAKDRVDC und IPGVPWCFKPLQEAECTF.

### Beispiel 3: Immunhistochemischer Nachweis von TFF3 in Tumorzellen.

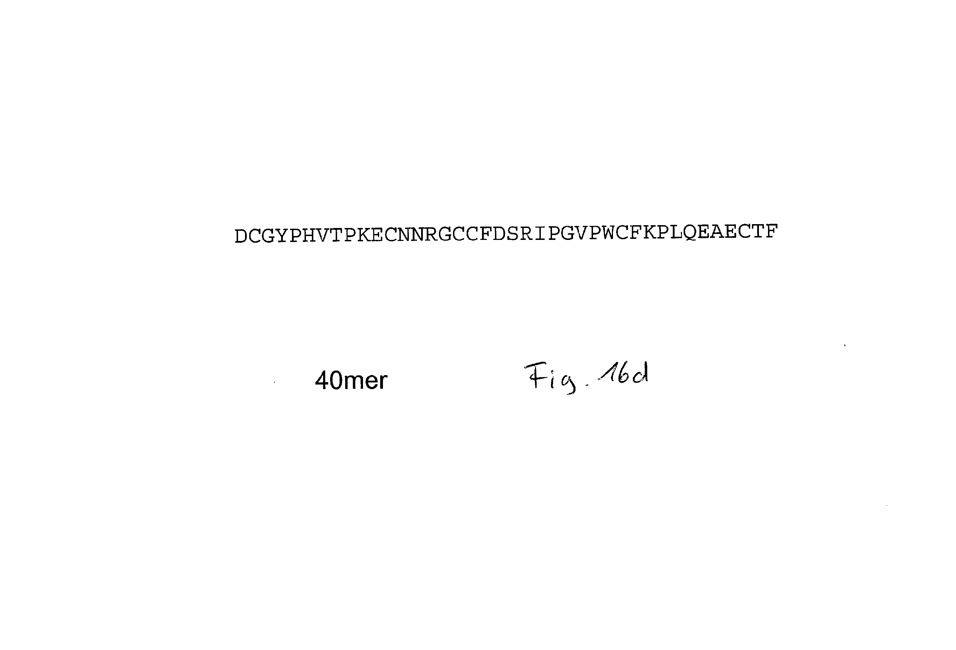

Primäre sowie lokal rezidive Tumoren werden aus den Patienten mit Uterus- und/oder Ovartumoren isoliert und als Paraffin bzw. Gefrierschnitte präpariert. Diese Schnitte werden mit einem anti-TFF3-Antikörper auf die Überexpression von TFF3 in Tumorzellen untersucht. Die immunhistologische Untersuchung mit dem TFF3-Antikörper zeigt höhere Expression von TFF3 in den Tumorzellen im Vergleich zu umliegenden Normalgewebe. Die Untersuchung erfolgt im Einzelnen durch Inkubation mit dem anti-TFF3 Antikörper als primärem Antikörper aus Ratte, einem biotinyliertem sekundären anti-Ratte Antikörper und einer Streptavidingekoppelten Meerrettichperoxidase. Die Färbung erfolgt mit DAB als chromogenen Substrat (braune Färbung). Die Gegenfärbung erfolgt mit Hemalaun-Lösung (blaue Färbung). Es sind maligne und nichtmaligne Zellen unterscheidbar, wobei die malignen Zellen eine starke Färbung, i.e. hohen TFF3 Gehalt, aufweisen, während die nichtmalignen Zellen nur moderat gefärbt sind. In der Figur 7 a-c sind Ergebnisse mit anti-TFF3 Antikörpern aus Ratte in zwei Ovarialkarzinomen (7 a,b) sowie einem Ovarialnormalgewebe (7 c) dargestellt (Parafinschnitte). Jeweils links sind die Schnitte mit anti-TFF3 Antikörper zu sehen während jeweils rechts zugeordnete Negativkontrollen mit einer Färbung mit Präimmunserum aus der Ratte dargestellt sind. Man erkennt, dass Tumorzellen, die TFF3 überexprimieren, eine starke rötliche Färbung zeigen.

Darüber hinaus kann die Untersuchung der Gewebeschnitte auch mittels Immunofluoreszenz erfolgen. Dafür wird der anti-TFF3 Antikörper entweder direkt mit einem Fluoreszenzfarbstoff markiert oder mit einem sekundären fluoreszenzmarkiertem Antikörper, der gegen die Spezies gerichtet ist, aus der der Primärantikörper gewonnen wurde. Die Auswertung erfolgt mittels Fluoreszenzmikroskopie bzw. mittels Laser Scanning Mikroskopie.

### Beispiel 4: RNA-Inhibitoren

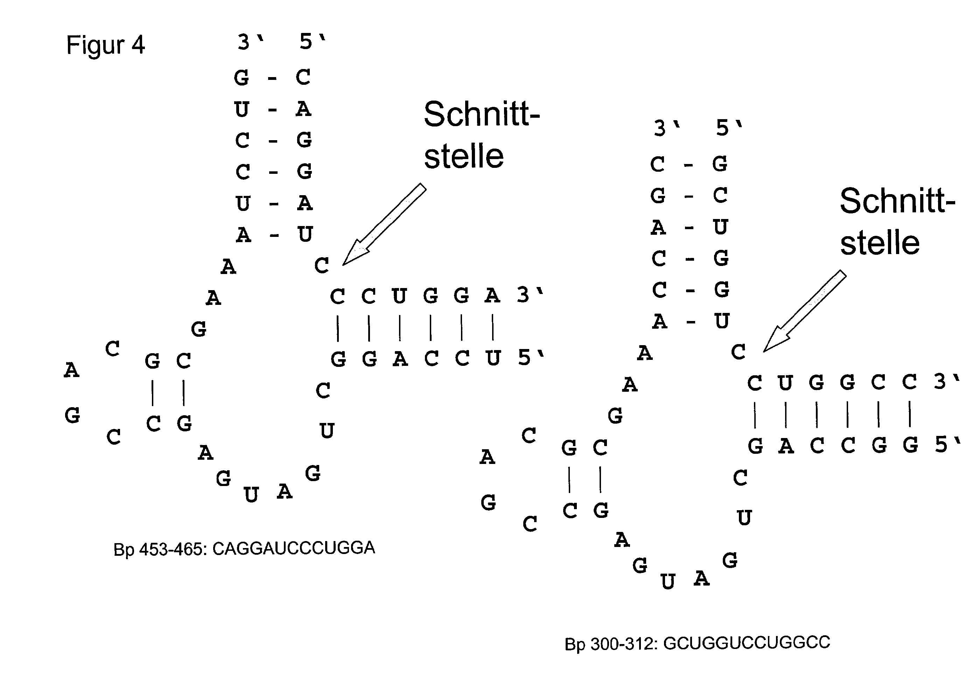

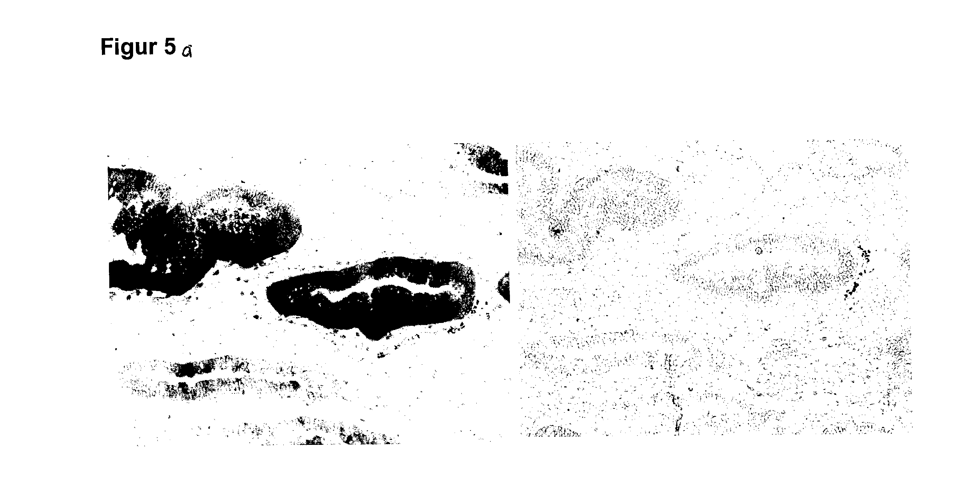

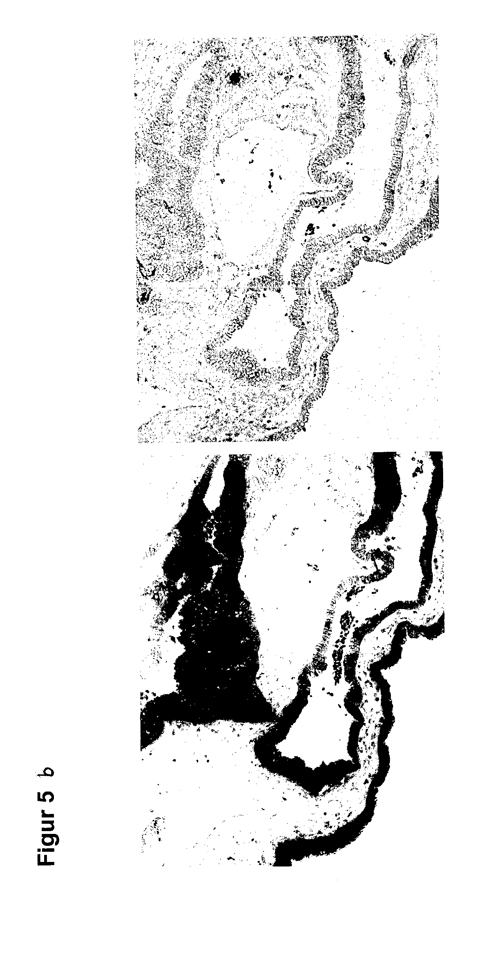

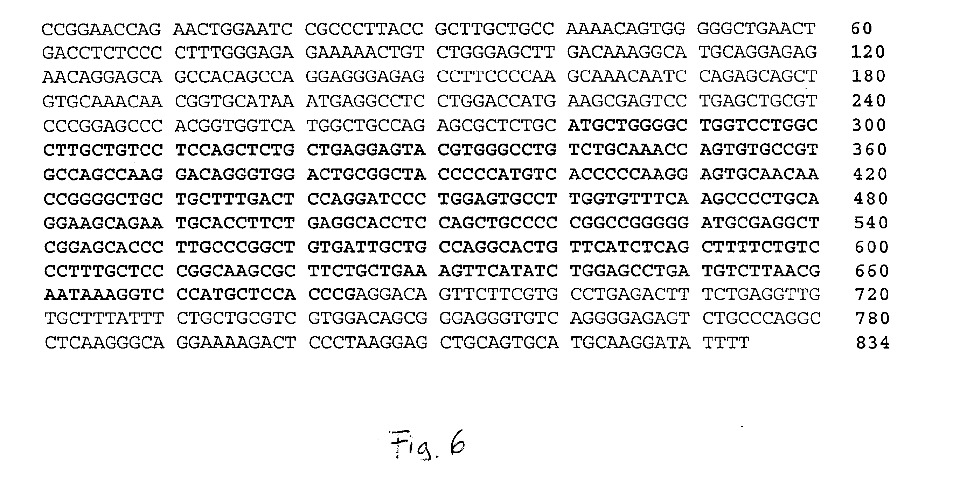

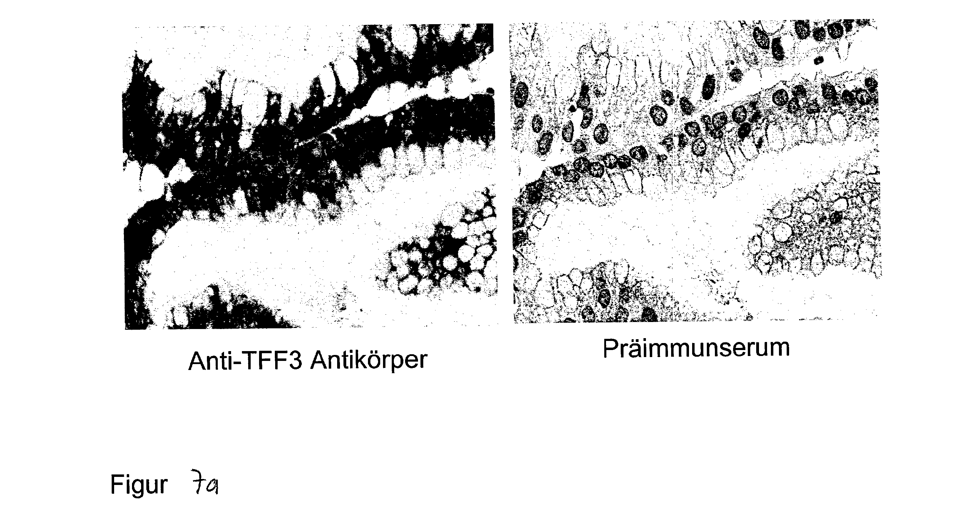

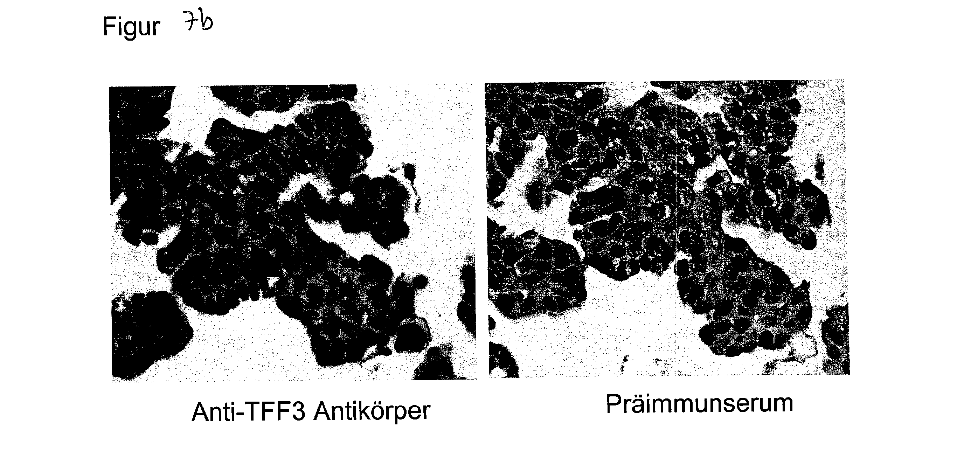

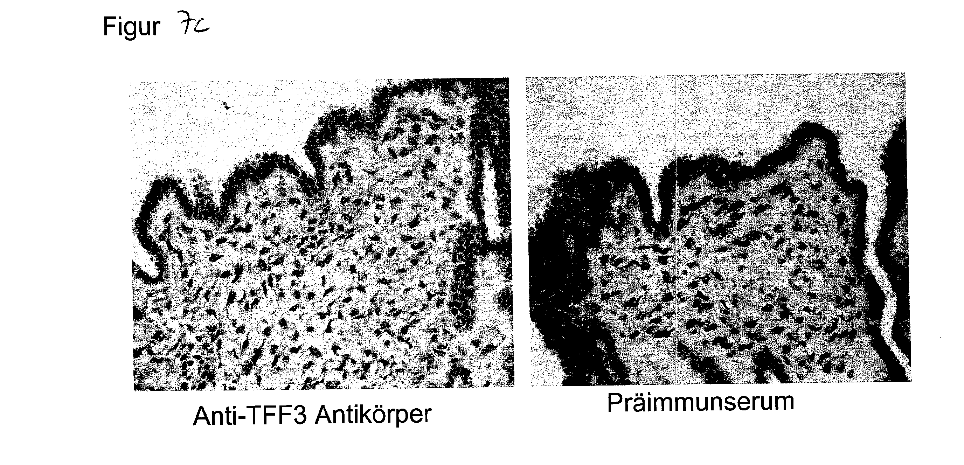

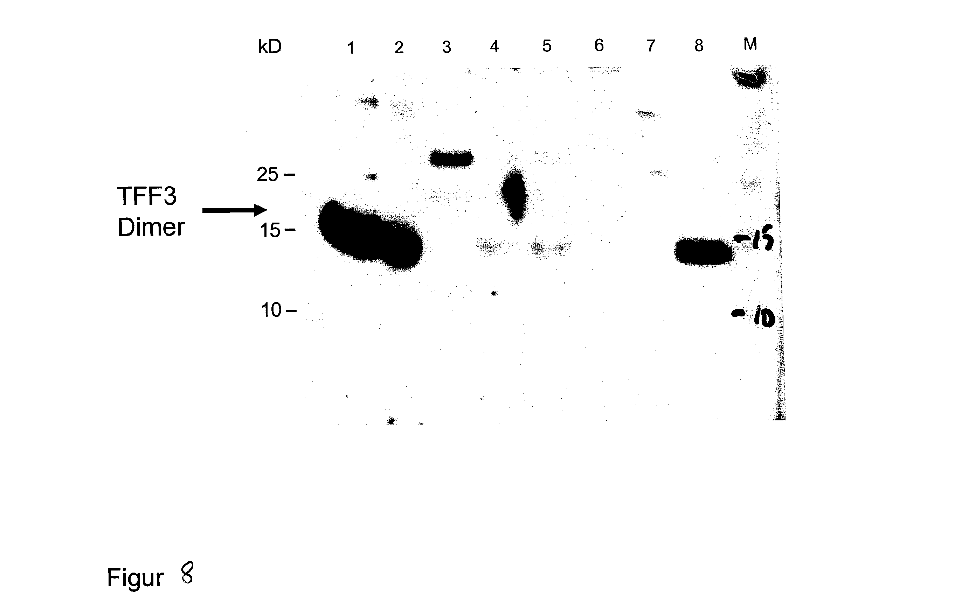

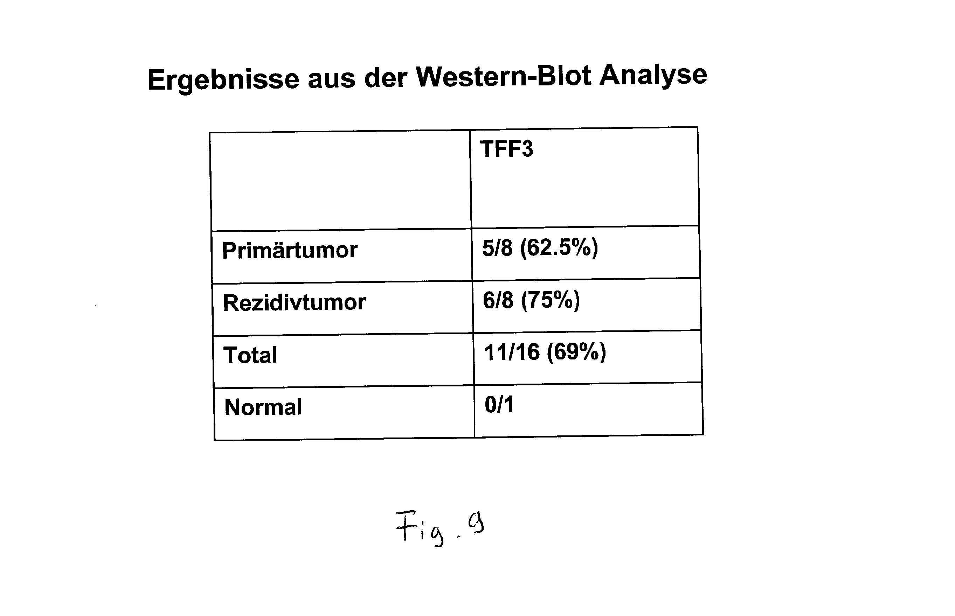

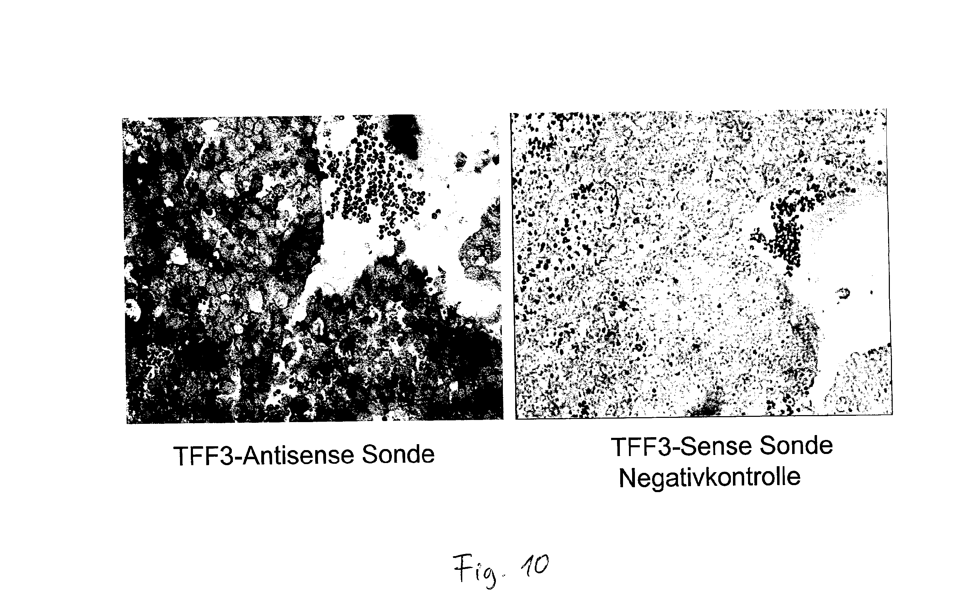

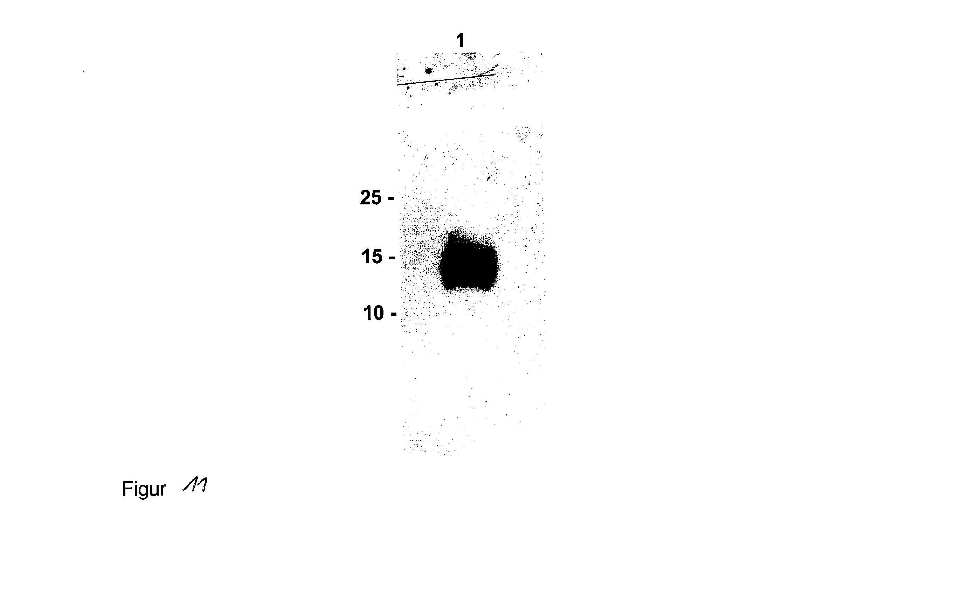

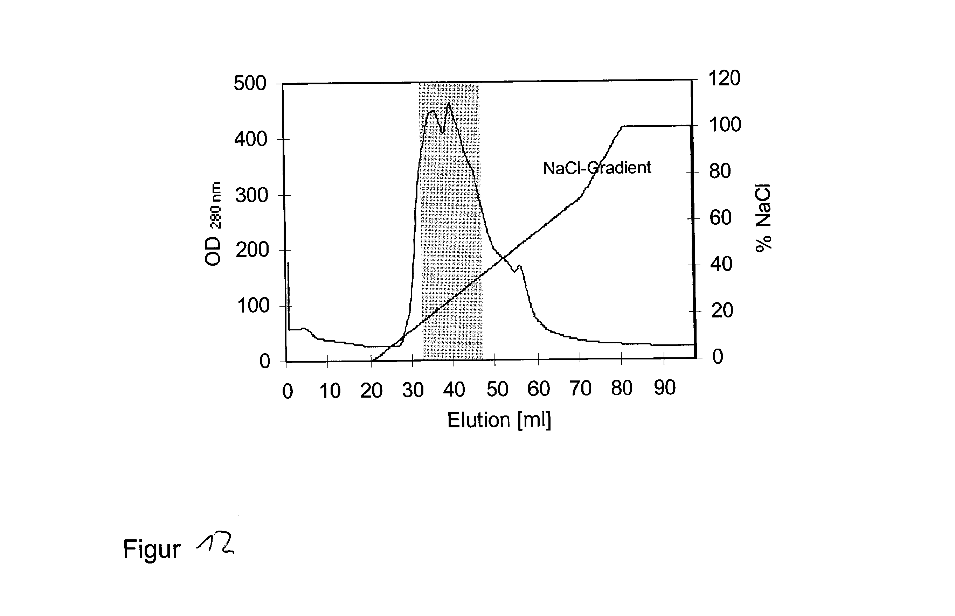

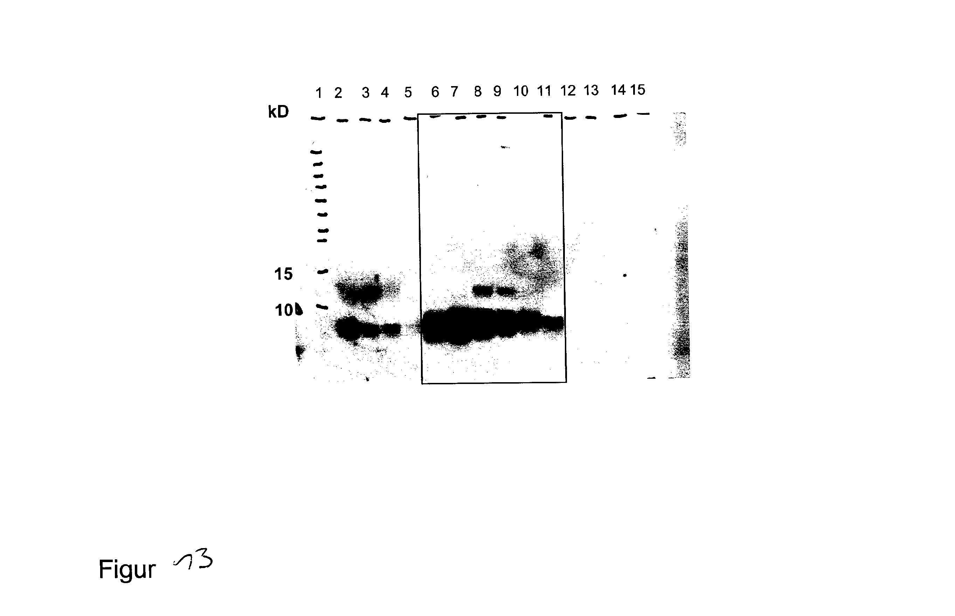

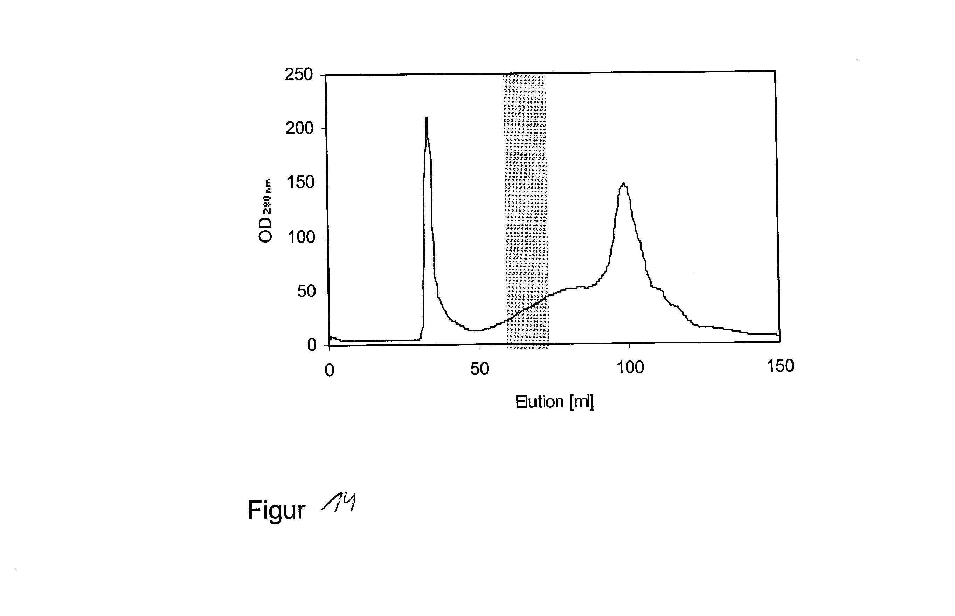

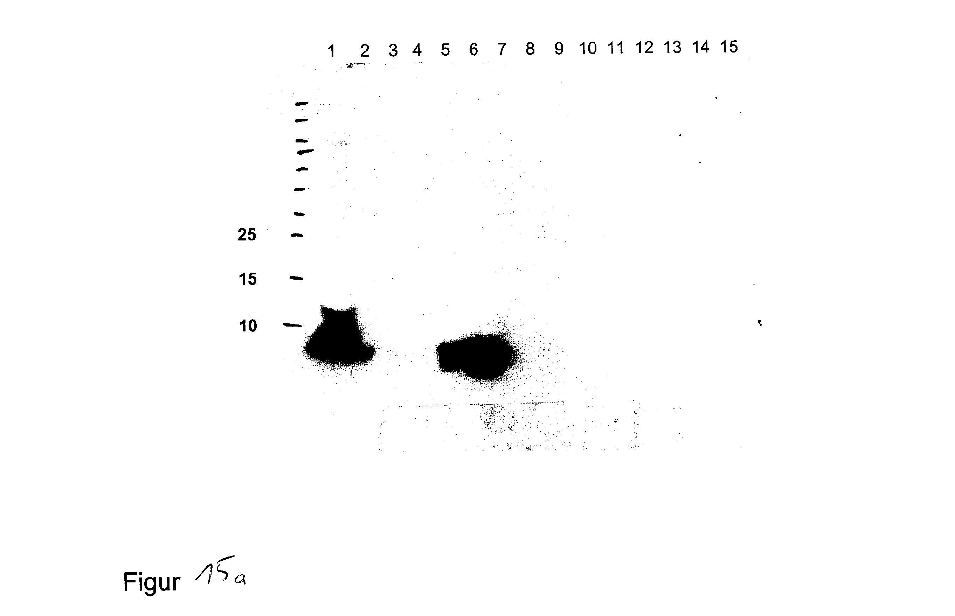

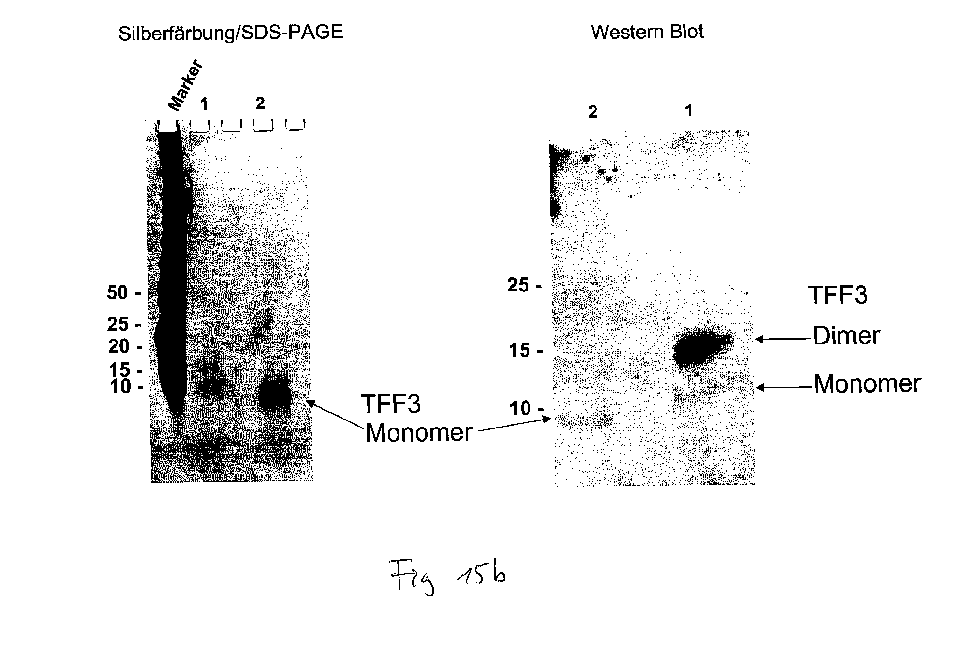

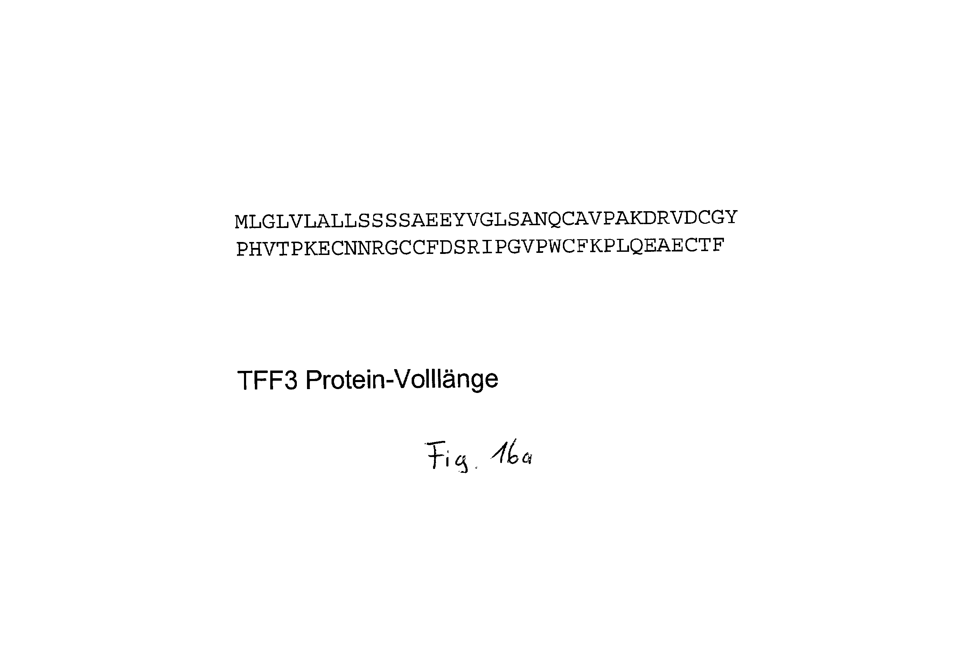

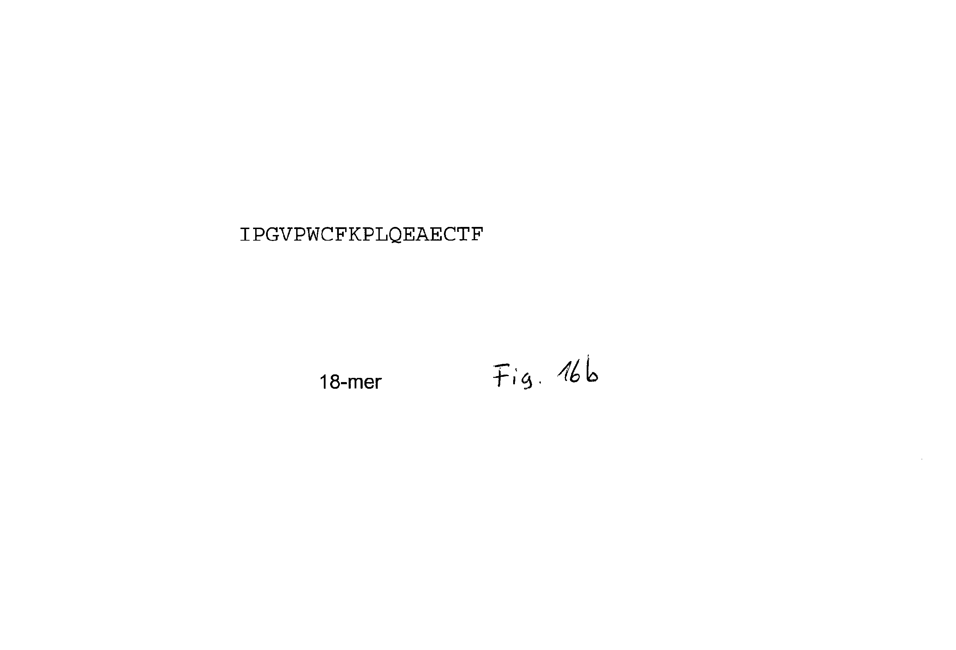

In der Figur 4 ist ein Hammerhead Ribozym dargestellt, die TFF3 an der dargestellten Stelle schneidet und so die Aktivität eventueller Translationsprodukte inhibiert oder zumindest reduziert. Als antisense Sequenz kommt beispielsweise GCT CTG CAT GCT GGG GCT GG in Frage.

### Beispiel 5: RNA in situ Hybridisierung

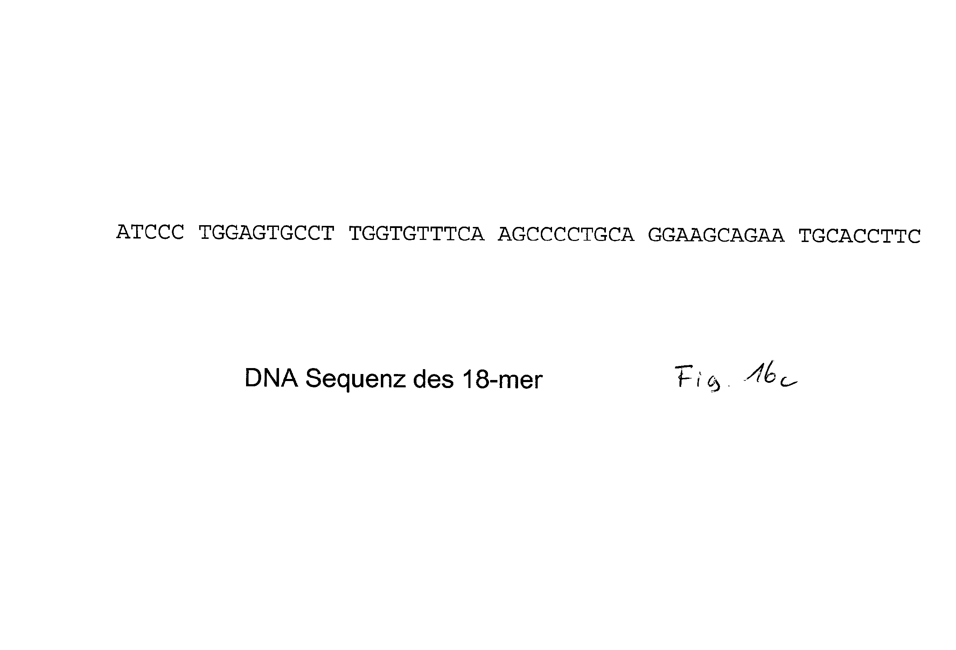

Gewebeproben aus Ovartumorgewebe wurde mit einer TFF3 spezifischen Antisense Sonde inkubiert und mit BM-Purple gefärbt. Die Gegenfärbung erfolgte mit Kernecht-Rot. Es waren maligne und nichtmaligne Epithelzellen unterscheidbar, wobei die malignen Zellen eine starke Anfärbung der RNA in der in situ Hybridisierung aufwiesen. In der Figur 5 sind die erhaltenen Schnitte dargestellt. Man erkennt, daß im Ovartumorgewebe TFF3 (=bt19) auch auf RNA Ebene überexprimiert ist. In Normalgewebe waren drei untersuchte Proben negativ. In 6 untersuchten Tumorgewebeproben waren 3 positiv (Färbung über mehr als 10% der Fläche), und zwar mit einem Grading von 2+ (1) und 3+ (2).

Darüber hinaus wurden Xenografts der Ovarialtumorzelllinie OV90, welche intraovarial bzw. orthotop in NMRI-Nacktmäuse injiziert wurde, mit einer TFF3-spezifischen Sonde inkubiert und mit BM-Purple gefärbt. Es erfolgte eine Gegenfärbung mit Kernecht-Rot. In der Figur 10 sind die erhaltenen Schnitte dargestellt. In den OV90 Xenografts ist erkennbar, dass TFF3 auch auf RNA Ebene in Xenografts überexprimiert ist. Tumorzellen zeigen eine starke Expression von TFF3 (dunkelbaue Färbung).

### Beispiel 6: proteinchemischer Nachweis von Tumorzellen.

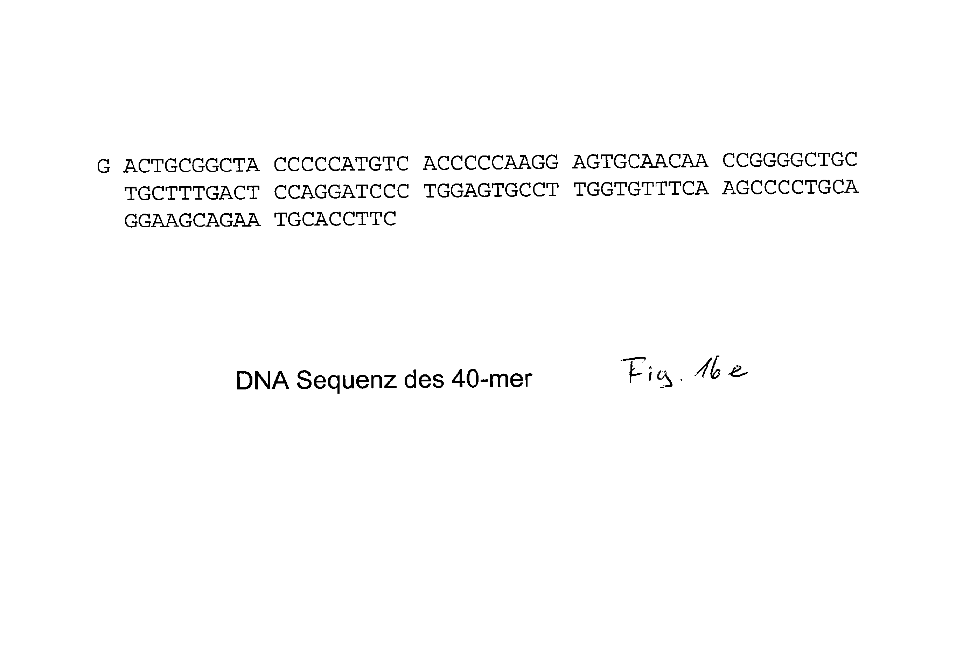

Primäre sowie lokal rezidive Tumoren wurden aus Patienten mit Uterus- und/oder Ovartumoren isoliert und als Gefrierschnitte präpariert. Aus ca. 20 Schnitten wurden mittels NP40 Puffer ein Proteinlysat hergestellt. Nach einer Proteinbestimmung wurden 30 µg/µl Protein/Laufbahn auf ein 16%-iges Tris-Glycin-Gel aufgetragen und eine SDS-PAGE unter nicht-reduzierenden Bedingungen durchgeführt. Mit TFF3-spezifischen Antikörpern aus Ratte oder Kannichen wurde auf dem Western-Blot eine 15 kD Bande, welche spezifisch für TFF3-Dimer ist, identifiziert. Die Ergebnisse sind in der Figur 8 dargestellt. Die Spuren 1 bis 5 sowie 7 und 8 sind aus Tumorzellen, die Spur 6 ist eine Negativkontrolle (Proteinlysat aus Normalgewebe des Ovars). Man erkennt, dass in den Lysaten von 5 der 7 aufgetragenen Ovartumorgeweben TFF3 auf Proteinebene exprimiert wird und in dimerisierter Form vorliegt. In der Figur 9 ist das Ergebnis der Analyse von 16 Ovartumorgeweben zusammengefasst.

### Beispiel 7: TFF3 Isolierung und Reinigung.

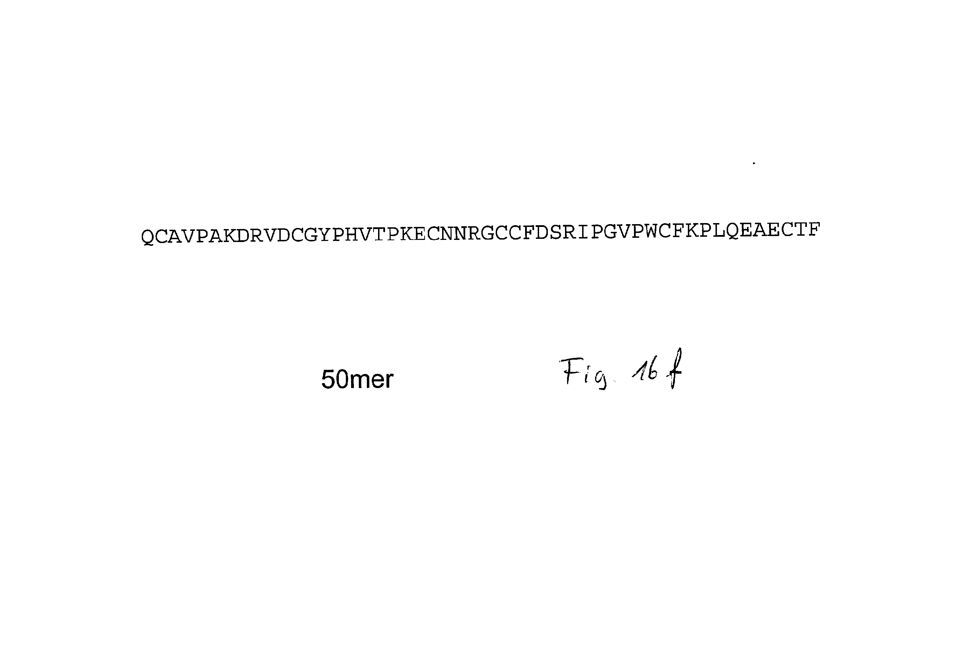

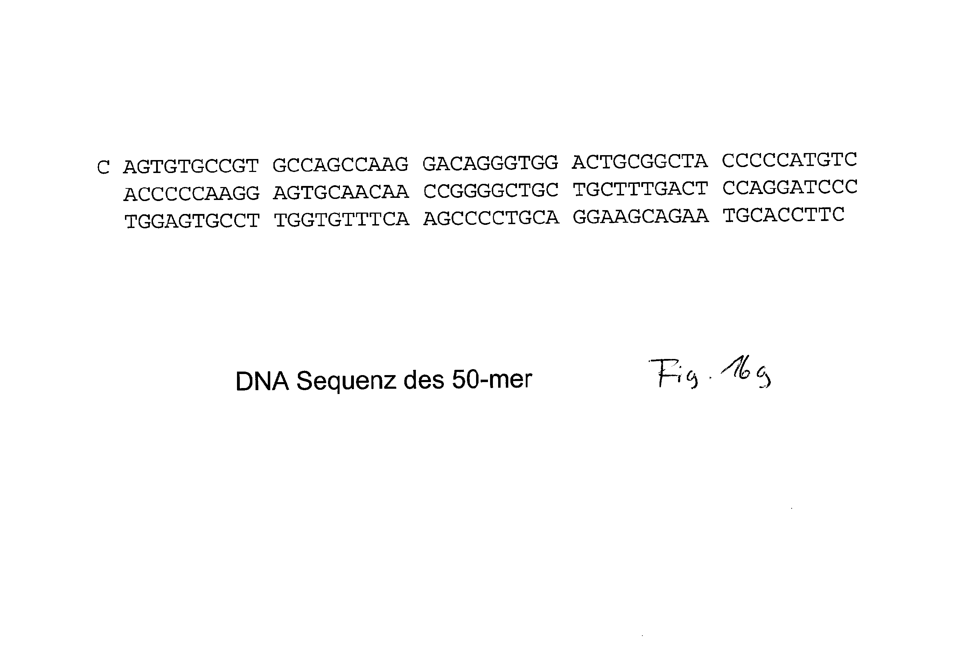

Volllängen TFF3 wurde in ein Baculovirus-Vektor kloniert. High Five Insektenzellen wurden anschließend mit den rekombinanten Baculoviren infiziert. Die Zellen wurden unter serumfreien Bedingungen kultiviert. 72 Stunden nach der Infektion wurde zu den Zellen eine Tablette Protease-Inhibitor Mix (Roche) pro 100 ml Zellkulturvolumen gegeben und weitere 24 Stunden kultiviert. Durch Zentrifugation wurde der Zellkulturüberstand gewonnen und gegen 10 mM Tripuffer pH 8,0 dialysiert. In Figur 11 ist ein Western Blot vom dialysierten Zellkulturüberstand, aufgetragen unter nicht reduzierenden Bedingungen, dargestellt (Detektion mit einem TFF3-spezifischen Antikörper aus der Ratte). Das TFF3 Protein liegt überwiegend als Dimer vor und zeigt die erwartete Grösse. Der dialysierte Überstand wurde anschließend über eine Q-Sepharose-Säule (Pharmacia) gegeben und mit ansteigender NaCl Konzentration bis 500 mM eluiert. TFF3 wurde bei einer NaCl-Konzentration zwischen 20 und 40 mM eluiert (Fig. 12). Fraktionen aus der Q-Sepharose-Säule wurden in einem Gel aufgetrennt und anschließend im Western Blot mit einem anti-TFF3 Antikörper aus dem Kaninchen detektiert (Fig. 13). Die Faktionen 6 - 11, die einen hohen Anteil an TFF3 aufweisen, wurden gepoolt und durch Ultrafiltration (Membran 3 kD cut off) aufkonzentriert. Die vereinigten, konzentrierten Fraktionen wurden über eine Biogel-30 Chromatographie-Säule gegeben und unter PBS Bedingungen eluiert (Fig. 14). Figur 15a zeigt den zugehörigen Western Blot. Die demgemäß TFF3 entahltenden Fraktionen wurden dann aufkonzentriert. Schließlich zeigt die Figur 15b ein Silber-gefärbtes SDS-PAGE (links) und Western Blot (rechts) von gereinigtem, aufkonzentriertem TFF3, aufgetragen unter nichtreduzierenden (jeweils Spur 1) und reduzierenden (jeweils Spur 2) Bedingungen. Man entnimmt dem Western Blot, dass das erhaltene TFF3 überwiegend als Dimer vorliegt, und dem Silbergel, dass keine detektierbaren Kontaminationen durch andere Proteine vorliegen. Im Western Blot ist das Monomer unter reduzierenden Bedingungen schwach detektierbar. Dies ist dadurch zu erklären, dass der Antikörper gegen die nicht reduzierte Form generiert wurde und deshalb eine schwächere Affinität zur reduzierten Form aufweist. Die Schwäche der Bande läßt keinen Rückschluss auf die Menge des Proteins zu.

### Beispiel 8: antiapoptotische Wirkung von TFF3.

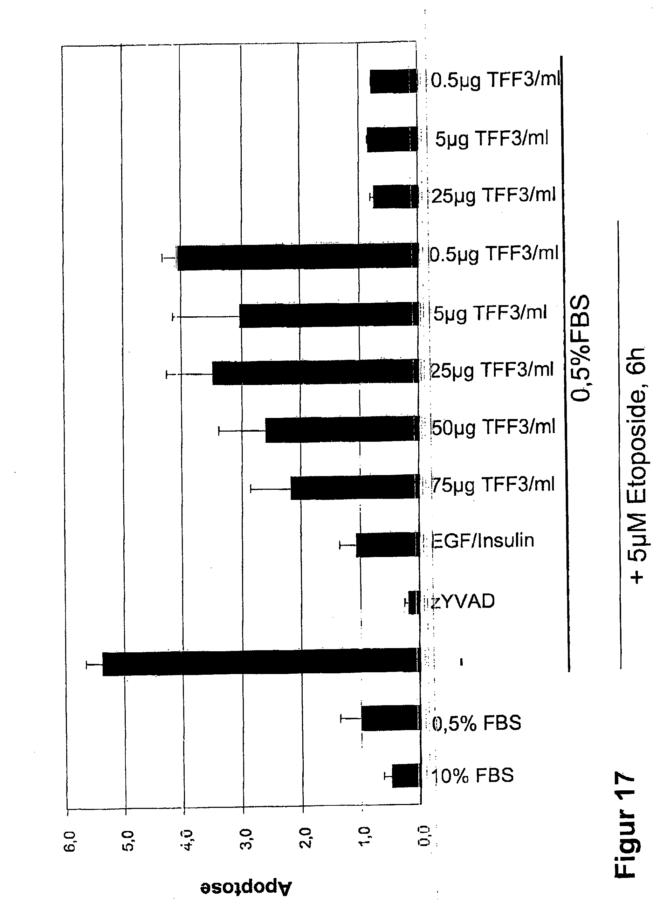

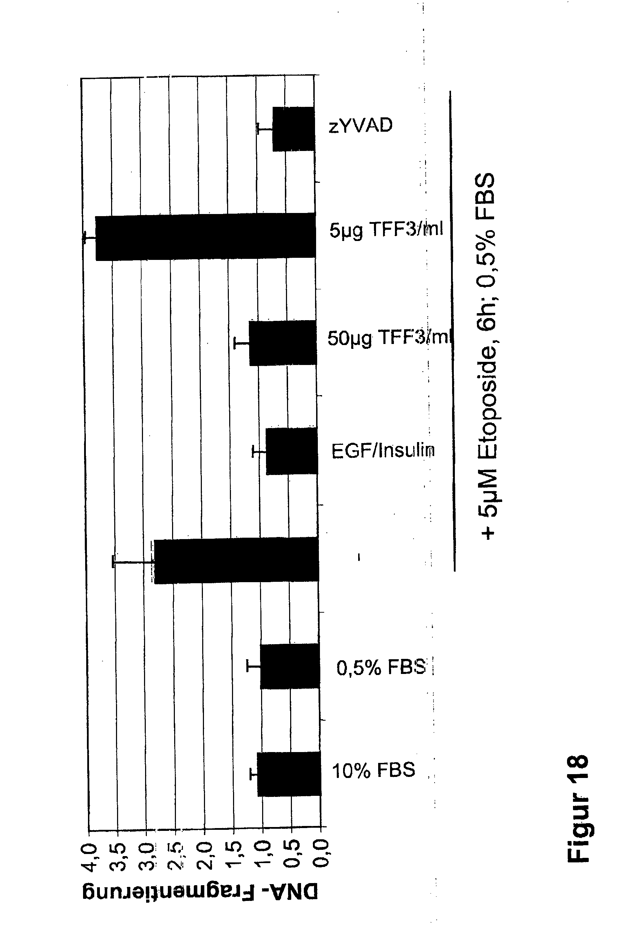

In den Figuren 17 und 18 sind Ergebnisse zu Experimenten dargestellt, durch welche die protektive Wirkung von Überexprimiertem TFF3 in Krebszellen belegt wird.

Figur 17 stellt Ergebnisse aus einem Apoptose Assay dar, bei welchem die Apoptosefaktoren Caspase-3 und Caspase-7 (Hauptenzyme im Apoptose-Signaltransduktionsweg) in AGS-Magenkarzinomzellen gemessen werden (Apo-ONE, erhältlich von der Firma Promega). Apoptose wurde durch Gabe von 5 µM Etoposide bei 6-stündiger Inkubation induziert. Es wurden zusätzlich verschiedene Dosen TFF3 eingesetzt. Die beiden linkseitigen Balken zeigen die Apoptoserate bei unbehandelten Zellen. Der dritte Balken von links zeigt, dass bei alleiniger Gabe von Etoposide die Apoptoserate drastisch erhöht ist. Die drei rechtesten Balken zeigen, dass alleinige Gabe von TFF3 nahezu keine Änderung der Apoptoserate bewirkt. Der vierte Balken von links zeigt, dass Zellen, die mit dem Caspaseinhibitor zYVAD behandelt wurden, auch bei Gabe von Etoposide eine drastisch reduzierte Apoptoserate aufweisen. Wesentlich gegenüber diesen Negativ- und Positivkontrollen ist nunmehr, dass bei gleichzeitiger Gabe der proapoptotischen Substanz Etoposide und von TFF3 die Apoptoserate mit steigender TFF3 Konzentration sinkt. Dies belegt, dass Überexpression von TFF3 in Krebszellen deren Apoptose verhindert bzw. reduziert, i.e. TFF3 protektiv wirkt. Dies bedeutet umgekehrt, dass eine Hemmung von TFF3 ursächlich in die krebsbedingt fehlregulierten natürlichen Apoptoseprozesse eingreift und das Absterben der Krebszellen fördert.

Die wird zusätzlich belegt durch die Ergebnisse der Figur 18. Hierin ist ein Apoptose Assay dargestellt, wobei die DNA-Fragmentierung in AGS-Magenkarzinomzellen nach Induktion mit Etoposide gemessen wird (Cell Death Detection ELISA, erhältlich von der Firma Roche Diagnostics). Man erkennt, dass zusätzlich TFF3 behandelte Zellen eine um ca. einen Faktor 2,2 verringerte DNA Fragmentierung aufweisen. Als Kontrollen wurde dieselben, wie vorstehend, eingesetzt.

## Patentansprüche

1. Verwendung einer für TFF3 codierenden Nukleinsäure und/oder eines TFF3 Peptids oder Proteins zur Detektion von Uterus- und/oder Ovartumoren oder zur Detektion eines Risikos der Erkrankung an Uterus- und/oder Ovartumoren, wobei eine Uterus- und/oder Ovar-Gewebeprobe auf Übertranskription von TFF3 RNA oder auf Überexpression eines TFF3 Proteins untersucht wird.

2. Verwendung nach Anspruch 1, wobei eine an für TFF3 codierende Nukleinsäure oder eine an TFF3 Protein oder Peptid bindende Detektorsubstanz, vorzugsweise enthaltend eine Reportergruppe, verwendet wird, wobei Bindung besagter Nukleinsäure und/oder besagten Proteins oder Peptids an die Detektorsubstanz halbquantitativ oder quantitativ detektiert wird.

3. Verwendung einer TFF3 RNA oder eines TFF3 Proteins oder Peptids oder einer TFF3 DNA, insbesondere mit regulatorischen Eigenschaften, zum Screenen nach daran bindenden Substanzen, insbesondere prospektiven Wirkstoffen zur Inhibierung von besagter RNA oder besagtem Protein oder Peptid oder prospektiven Detektorsubstanzen, wobei eine prospektive Substanz oder eine Mischung solcher prospektiver Substanzen mit besagter RNA oder besagtem Protein oder Peptid kontaktiert wird, wobei mit einem Bindungsassay Bindungsereignisse festgestellt werden, und wobei eine bindende prospektive Substanz, ggf. nach Dekonvolutierung, als zum Nachweis oder Behandlung von Uterusund/oder Ovartumoren geeignete Substanz selektiert wird.

4. Verwendung einer TFF3 inhibierenden oder daran bindenden Substanz zur Herstellung einer pharmazeutischen Zusammensetzung zur Behandlung von Uterus- und/oder Ovartumoren.

5. Verwendung nach Anspruch 4, wobei die Substanz ein Antikörper ist, welcher durch Immunisierung eines nicht-menschlichen Säugetiers mit einem TFF3 Peptid oder Protein, oder mit cDNA von TFF3 transfizierten NIH3T3-Zellen, oder mit endogen TFF3 expromierenden Tumorzellen, oder mit in Insektenzellen hergestelltem TFF3 erhältlich ist, oder ein Phage-Display Antikörper ist.

6. Verwendung nach Anspruch 4, wobei die Substanz eine Mimikryverbindung eines Antikörpers gegen ein TFF3 Peptid oder Protein ist.

7. Verwendung nach Anspruch 4, wobei die Substanz, ein Aptamer, eine antisense RNA, eine inhibitorische RNA, eine RNAi, oder ein Ribozym gegen TFF3 ist.

8. Verwendung nach einem der Ansprüche 4 bis 7, wobei die Substanz zusätzlich eine zytotoxische, radioaktive und/oder immunstimulierende Komponente trägt.

9. Verwendung nach einem der Ansprüche 4 bis 8, wobei die pharmazeutische Zusammensetzung zur lokalen Applikation in Tumorzellen enthaltendem Gewebe hergerichtet ist.

10. Verfahren zur Diagnose einer Uterus- und/oder Ovartumorerkrankung, wobei eine Detektorsubstanz in einer Ausführungsform mit einer Reportergruppe in zu untersuchendes Gewebe appliziert wird, wobei das zu untersuchende Gewebe dann einer Detektionsverfahrenstufc unterworfen wird, welche sensitiv für die Reportergruppe ist, und wobei im Fall der Detektion eines definierten Mindestwertes der Reportergruppe im Gewebe das Gewebe als Tumorzellen enthaltend qualifiziert wird.

11. Verfahren zur Behandlung einer Uterus- und/oder Ovartumorerkrankung, wobei eine pharmazeutische Zusammensetzung nach einem der Ansprüche 4 bis 9 in einer physiologisch wirksamen Dosis einem Patienten dargereicht wird.

12. Isoliertes Protein oder Peptid oder isolierte Nukleinsäure enthaltend eine Sequenz gemäß einer der Figuren oder bestehend hieraus, wobei das Protein oder Peptid oder die Nukleinsäure TFF3 funktional ist, nicht jedoch die fettgedruckte Teilsequenz aus Figur 6.

13. Verwendung oder Verfahren nach einem der Ansprüche 1 bis 11, wobei ein Protein oder Peptid oder eine Nukleinsäure nach Anspruch 12 verwendet wird.
